Europäisches Patentamt

European Patent Office

Office européen des brevets

(11) Veröffentlichungsnummer: **0 438 749 A1**

# EUROPÄISCHE PATENTANMELDUNG

(21) Anmeldenummer: 90125017.5

(22) Anmeldetag: **20.12.90**

(51) Int. Cl.5: **C09J 189/00, A61L 15/32**

(30) Priorität: **22.01.90 DE 4001714**

(43) Veröffentlichungstag der Anmeldung:
**31.07.91 Patentblatt 91/31**

(84) Benannte Vertragsstaaten:
**AT DE ES FR IT NL**

(71) Anmelder: **Beiersdorf Aktiengesellschaft**
**Unnastrasse 48**
**W-2000 Hamburg 20(DE)**

(72) Erfinder: **Pietsch, Hanns, Dr.**
**Mittelweg 25**
**W-2000 Hamburg 13(DE)**
Erfinder: **Wesselkamp, Ingrid**
**Krögerstrasse 54**
**W-2000 Hamburg 73(DE)**
Erfinder: **Borgschulte, Katrin, Dr.**
**Eppendorfer Weg 168**
**W-2000 Hamburg 20(DE)**

(54) **Selbstklebemassen auf Gelatinebasis.**

(57) Druckempfindliche Selbstklebemasse, enthaltend vernetzte Gelatine, einen organischen Weichmacher und $CaCl_2$ und/oder $ZnCl_2$.

EP 0 438 749 A1

EP 0 438 749 A1

## SELBSTKLEBEMASSE AUF GELATINEBASIS

Die vorliegende Erfindung betrifft druckempfindliche Selbstklebemassen.

Druckempfindliche Selbstklebemassen an sich sind seit langem bekannt. Sie werden beispielsweise in medizinischen Pflastern, Klebebändern und Etiketten verwendet. Die wichtigsten Selbstklebemassen sind:
* Kautschukklebemassen (Natur- oder Kunstkautschuke)
* Acrylatklebemassen (Acrylester- oder Methacrylester(co-)polymerisate)
* polyurethanselbstklebemassen
* Silikonselbstklebemassen.

Jedes dieser Systeme hat charakteristische Eigenschaften und wird für bestimmte Anwendungen bevorzugt.

Als Selbstklebemasse für Pflaster werden Kautschuk- und Acrylatselbstklebemassen verwendet.

Kautschukselbstklebemassen haften sehr gut auf der menschlichen Haut, jedoch verursachen die notwendig darin enthaltenen Klebeharze häufig allergische Reaktionen. Acrylatselbstklebemassen rufen nur sehr selten Allergien hervor, haben aber den Nachteil, daß sie zwar auf trockenen Flächen gut, auf feuchten Flächen, z.B. verschwitzter Haut, jedoch schlecht kleben.

Es ist bekannt, daß man auf Basis von Gelatine wasseraktivierbare Klebmassen herstellen kann, wie man sie beispielsweise für Briefmarken verwendet. Diese Massen werden erst nach Befeuchten klebrig, büßen aber nach dem Antrocknen ihre Klebkraft vollständig ein.

In EP-A-0 018 093 werden Selbstklebemassen unter der Bezeichnung "gelatinous tacky articles" beschrieben. Gelatine ist aber nur ein Nebenbestandteil. Bekannte wasserlösliche Klebrigmacher, Polysaccharidharze wie Tragacanth oder Gummi arabicum, verleihen diesen Massen Klebrigkeit.

Es ist auch bekannt, Gelatine zu vernetzen. In EP-A-0 097 846 wird beschrieben, wie Gelatine in fester Form als Pulver, Schuppen oder Blatt in einer Zwei-Phasen Reaktion mit Vernetzern wie Formaldehyd, Glyoxal, Glutarsäuredialdehyd, Dicarbonsäurechloriden und/oder Diisocyanaten umgesetzt wird. Selbstklebemassen erhält man auf diese Weise nicht.

Es war also die Aufgabe der vorliegenden Erfindung, druckempfindliche Selbstklebemassen ohne die Nachteile der herkömmlichen Produkte zu entwickeln. Insbesondere sollten die erfindungsgemäßen Selbstklebemassen als Klebemasse für medizinische Pflaster, aber auch als Klebemasse für allgemeine Zwecke, z.B. Etiketten und Klebebänder, geeignet sein.

Es wurde gefunden, und darin liegt die Lösung der Aufgabe, daß eine Selbstklebemasse, enthaltend vernetzte Gelatine, einen organischen Weichmacher und $CaCl_2$ und/oder $ZnCl_2$, die gewünschten Eigenschaften besitzt.

Überraschend ist insbesondere, daß es diese beiden anorganischen Salze sind, die aus einer nichtthixotropen Masse wie vernetzter Gelatine einen Klebstoff mit erstaunlichen Eigenschaften machen.

So erhält man aus weichgemachter, vernetzter Gelatine ohne diese Salze biegsame Filme, die sich je nach Weichmachergehalt mehr oder weniger fettig, nicht jedoch klebrig anfühlen. Beträgt der Gehalt an $CaCl_2$ oder $ZnCl_2$ weniger als 10 Gew.-% , erhält man keine Selbstklebemasse. Bei einem Gehalt an $CaCl_2$ oder $ZnCl_2$ oberhalb von 45 Gew.-% trocknet die Masse schlecht und hat keine ausreichende Kohäsion.

Die erfindungsgemäßen Selbstklebemassen bestehen vorzugsweise aus

```
25  - 50   Gew.-% Gelatine

15  - 45   Gew.-% Weichmacher

10  - 40   Gew.-% CaCl₂ und/oder ZnCl₂

0,1 - 5,0  Gew.-% Vernetzer,
```

bezogen auf die wasserfreie Substanz.

Die fertigen Selbstklebemassen enthalten etwa 10 - 20 Gew.-% Wasser, bezogen auf das Gesamtgewicht der Zusammensetzung. Wenn in dieser Schrift Gewichtsprozente angegeben sind, sind diese immer auf die wasserfreie Substanz bezogen, sofern nicht anders angegeben.

Die erfindungsgemäßen Selbstklebemassen können große Mengen Wasser aufnehmen und quellen, ohne sich aufzulösen Gleichwohl sind die erfindungsgemäßen Klebemassen rückstandsfrei mit fließendem

2

EP 0 438 749 A1

Wasser entfernbar.

Die Klebmassen sind besonders geeignet für Pflaster und Wundauflagen, aber auch für Klebebänder, Etiketten und dergleichen.

Im Gegensatz zu bisher bekannten Selbstklebemassen, die nur auf trockenen Substraten haften, haften die erfindungsgemäßen Selbstklebemassen umso besser, je feuchter der Untergrund ist. Sie sind hervorragend haut- und blutverträglich.

Die erfindungsgemäßen Selbstklebemassen können mehr als 100 Gew.-% Wasser, bezogen auf wasserfreie Masse, aufnehmen. Mit derartigen Klebemassen beschichtete Träger, beispielsweise medizinische Pflaster, lassen sich auch nach mehreren Stunden Verweildauer vom Untergrund, z.B. der Haut, rückstandsfrei wieder abziehen.

Die Gelatine bildet die Matrix der erfindungsgemäßen Selbstklebemassen. Als Abbauprodukt des Kollagens kann sie je nach dem Grad des Abbaus recht unterschiedliche Molekulargewichte aufweisen. In Abhängigkeit davon besitzen die verschiedenen Gelatinesorten unterschiedliche Gallertfestigkeiten, die üblicherweise in sogenannten "Bloomgraden" angegeben werden.

Die Gallertfestigkeit wird ermittelt, indem eine Gelatineplatte, die 6 2/3 Gew.-% luftgetrocknete (also noch feuchte) Gelatine enthält und 10 Stunden bei 18 °C gestanden hat, mit einem Stempel von 1/2 Zoll Durchmesser belastet wird. Dabei dringt der Stempel in die Gelatine ein. Die Gelatine hat die Gallertfestigkeit von 1 Bloomgrad, wenn bei einer Eindringtiefe von 4 mm ein Gewicht von 1 Gramm auf dem Stempel lastet.

Zur Herstellung der erfindungsgemäßen Selbstklebemassen sind alle handelsüblichen Gelatinesorten mit Gallertfestigkeiten von 30 - 300 Bloomgraden geeignet.

Wichtig für ausreichende Kohäsion und damit für rückstandsfreies Abziehen von der Unterlage ist eine ausreichende Vernetzung der Gelatine.

Gelatine enthält einen beträchtlichen Anteil an Hydroxyprolin und damit reaktionsfähige Hydroxylgruppen. Die OH-Zahl kann je nach Herkunft (Rind, Kalb, Schwein, Knochen, Haut, usw.) zwischen 30 und 150 mg KOH pro Gramm Gelatine betragen. Diese Hydroxylgruppe ist eine günstige Angriffsstelle für die Vernetzung. Es zeigte sich, daß selbst Gelatinen mit geringen OH-Zahlen ausreichend gut vernetzt werden können.

Die erfindungsgemäßen Vernetzer sind bevorzugt Alkoxysilylverbindungen, besonders bevorzugt gewählt aus der Gruppe der Verbindungen der folgenden allgemeinen Formel:

$HRN-(CH_2-CH_2-NR')_n-(CH_2)_m-Si(OR'')_3$

mit R, R' gewählt aus der Gruppe H, Methyl oder Ethyl,

und R'' gewählt aus der Gruppe Methyl, Ethyl

wobei n Werte von 0 - 5

und m Werte von 1 - 4 annehmen kann,

wie beispielsweise

3-Aminopropyltriethoxysilan,

N-Aminoethylaminopropyltrimethoxysilan, "Triaminosilan" $(H_2N-CH_2-CH_2-NH-CH_2-CH_2-NH-CH_2-CH_2-CH_2-Si(OCH_3)_3)$,

N-Methyl-3-aminopropyltrimethoxysilan.

Günstig sind aber auch

3-Ureidopropyltriethoxysilan,

3-(4, 5-Dihydroimidazol-1-yl)-propyltriethoxysilan,

3-Methacryloxypropyltriethoxysilan,

3-Glycidyloxypropyltrimethoxysilan, Vinyltriethoxysilan,

Vinyltrimethoxysilan, Tetramethoxysilan oder

Tetraethoxysilan.

Von den genannten Verbindungen hat sich das 3-Aminopropyltriethoxysilan als Vernetzer mit besonders guter physiologischer Verträglichkeit herausgestellt.

Die Alkoxysilylverbindungen sind unter Hydrolyse löslich, so daß sie sich gut in wäßrigen Systemen verarbeiten lassen. Selbstverständlich sind auch andere Vernetzer geeignet, vor allem solche, die mit Hydroxylgruppen reagieren können. Das ist besonders dann günstig, wenn die erfindungsgemäßen Selbstklebemassen für technische Anwendungen gedacht sind und die physiologische Verträglichkeit nachrangig ist. Insbesondere sind mehrfunktionelle Isocyanatverbindungen als Vernetzer vorteilhaft, von diesen besonders die aliphatischen Diisocyanate, ganz besonders bevorzugt das Hexamethylendiisocyanat.

Besonders wenn die Vernetzer wasserunlöslich sind, kann es günstig sein, einen Emulgator zuzugeben. Dieser kann grundsätzlich frei gewählt werden.

Eine besonders unproblematische Reaktionsführung und hochwertige Endprodukte werden jedoch

3

erreicht, wenn man Laurylsulfat, vorteilhaft in Mengen von 0,001 - 10,0 Gew.-% , bezogen auf das Trockengewicht der Zusammensetzungen, als Emulgator wählt.

Die erfindungsgemäßen Selbstklebemassen enthalten einen organischen Weichmacher oder eine Kombination mehrerer organischer Weichmacher, vorzugsweise bis zu 45 Gew.-% . Alle Weichmacher für unvernetzte Gelatine können auch in der vorliegenden Erfindung eingesetzt werden.

Die Auswahl der Weichmacher kann der Verwendung angepaßt werden. Für ein medizinisches Pflaster wird man bevorzugt eine physiologisch unbedenkliche Substanz wählen. Die Auswahl ist weniger kritisch, wenn die Klebemassen zur Beschichtung von Klebebändern dienen sollen. Besonders geeignet sind flüssige Polyole. Dabei ist das bevorzugte Polyol mit der geringsten Zelltoxizität das Glycerin.

Andere Polyole, die an sich eine gewisse Zelltoxizität aufweisen, können ebenfalls vorteilhaft eingesetzt werden, z.B., wenn sie besser verträglich mit den Selbstklebemassen sind, wie etwa Ethylenglycol, flüssige Polyethylenglycole verschiedener Molmasse, Propylenglycol-1,2, flüssige Polypropylenglycole-1,2 verschiedener Molmasse, Propylenglycol-1,3, flüssige Polypropylenglycole-1,3 verschiedener Molmasse, oder 2-Ethyl-2 (hydroxymethyl)-1, 3-propandiol.

Auch Zitronensäuretriethyl- oder trimethylester, Milchsäuremethyl- oder -ethylester, Glycolsäuremethyl-loder -ethylester sind vorteilhafte Weichmacher. Gut geeignet sind auch verzweigte Polyole.

Alle genannten Weichmacher vermischen sich homogen mit der Gelatine und schwitzen nicht aus.

Die erfindungsgemäßen Selbstklebemassen werden vorzugsweise hergestellt, indem die Gelatine und das $CaCl_2$ und/oder das $ZnCl_2$ mit Wasser versetzt und unter gleichzeitigem Rühren und Erwärmen auf ca. 45 - 95° C in Lösung gebracht werden. Sodann wird der Weichmacher hinzugegeben. In dieser Zusammensetzung ist die Masse in der Wärme unter Rühren mehrere Tage verarbeitungsfähig. Der oder die Vernetzer sollten erst kurz vor der weiteren Verarbeitung hinzugegeben werden.

Mit den erfindungsgemäßen Selbstklebemassen können flächige Träger, beispielsweise Gewebe (wie Baumwolle, Polyestergewebe, Polyamidgewebe, Zellwollgewebe), Vliese (wie Polyethylenvlies), Papier oder Folien (wie Polyesterfolie, PVC-Folie oder sonstige Hart- oder Weichfolien) beschichtet werden.

Vorteilhaft werden die Träger zwischen 45 und 95° C auf einer Gießmaschine im Walz- oder Rakelauftrag beschichtet. Werden saugfähige Träger (wie Gewebe, Papier oder Vlies) verwendet, wird ein indirektes Auftragsverfahren bevorzugt. Dabei wird die Selbstklebemasse zunächst auf einen nicht saugfähigen klebstoffabweisenden Hilfsträger gestrichen, auf diesem geliert und getrocknet und dann auf den endgültigen Träger überkaschiert. Masseaufträge von 10 - 200 g/mm² haben sich als besonders günstig herausgestellt.

Die folgenden Beispiele sollen die vorliegende Erfindung näher erläutern, ohne daß aber beabsichtigt ist, die Erfindung auf diese Beispiele zu beschränken.

Beispiel 1

15 Gewichtsteile Gelatine (enthaltend 10 Gew.-% Wasser), 10 Gewichtsteile Glycerin (enthaltend 15 Gew.-% Wasser), 10 Gewichtsteile $CaCl_2$ (enthaltend 2 Gew.-% Wasser) wurden in 64,8 Gewichtsteile Wasser gegeben und sodann unter Rühren auf 55° C gebracht bis eine homogene Lösung entstanden war. In diese Lösung wurden 0,2 Gewichtsteile 3-Aminopropyltrisethoxysilan hineingerührt. Diese Lösung wurde auf eine ebene polierte Teflonplatte gegossen, so daß nach 48 Stunden bei 22° C und 40% relativer Feuchte eine Masse-Schicht von 100 - 120 g/m² übrig blieb.

Diese Schicht wurde sodann auf ein Textil-Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 2

20 Gewichtsteile Gelatine (enthaltend 10 Gew.-% Wasser), 10 Gewichtsteile Ethylenglycol, 5 Gewichtsteile $CaCl_2$ (enthaltend 2 Gew.-% Wasser) wurden in 64,7 Gewichtsteile Wasser gegeben und sodann unter Rühren auf 55° C gebracht bis eine homogene Lösung entstanden war. In diese Lösung wurden 0,3 Gewichtsteile 3-Aminopropyltrisethoxysilan hineingerührt. Diese Lösung wurde auf eine ebene polierte Teflonplatte gegossen, so daß nach 48 Stunden bei 22° C und 40% relativer Feuchte eine Masse-Schicht von 100 - 200 g/m² übrig blieb.

Diese Schicht wurde sodann auf ein Textil-Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 3

15 Gewichtsteile Gelatine (enthaltend 10 Gew.-% Wasser), 10 Gewichtsteile Propylenglycol-1,2 , 6 Gewichtsteile $CaCl_2$ (enthaltend 2 Gew.-% Wasser) wurden in 68,5 Gewichtsteile Wasser gegeben und sodann unter Rühren auf 60° C gebracht bis eine homogene Lösung entstanden war. In diese Lösung wurden 0,5 Gewichtsteile 3-Aminopropyltrisethoxisilan hineingerührt. Diese Lösung wurde auf eine ebene polierte Teflonplatte gegossen, so daß nach 48 Stunden bei 22° C und 40% relativer Feuchte eine Masse-Schicht von 80-100 $g/m^2$ übrig blieb.

Diese Schicht wurde sodann auf ein Textil-Gewebe mit einem Gewicht von 120 $g/m^2$ überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 4

15 Gewichtsteile Gelatine (enthaltend 10 Gew.-% Wasser), 8 Gewichtsteile Propylenglycol-1,3 , 5 Gewichtsteile $CaCl_2$ (enthaltend 2 Gew.-% Wasser) wurden in 71,5 Gewichtsteile Wasser gegeben und sodann unter Rühren auf 60° C gebracht bis eine homogene Lösung entstanden war. In diese Lösung wurden 0,5 Gewichtsteile 3-Aminopropyltrisethoxysilan hineingerührt. Diese Lösung wurde auf eine ebene polierte Teflonplatte gegossen, so daß nach 48 Stunden bei 22° C und 40% relativer Feuchte eine Masse-Schicht von 100-120 $g/m^2$ übrig blieb.

Diese Schicht wurde sodann auf ein Textil-Gewebe mit einem Gewicht von 120 $g/m^2$ überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 5

15 Gewichtsteile Gelatine (enthaltend 10 Gew.-% Wasser), 5 Gewichtsteile Ethylendiglycol 400 , 3 Gewichtsteile $CaCl_2$ (enthaltend 2 Gew.-% Wasser) wurden in 76,7 Gewichtsteile Wasser gegeben und sodann unter Rühren auf 60° C gebracht bis eine homogene Lösung entstanden war. In diese Lösung wurden 0,3 Gewichtsteile 3-Aminopropyltrisethoxysilan hineingerührt. Diese Lösung wurde auf eine ebene polierte Teflonplatte gegossen, so daß nach 48 Stunden bei 22° C und 40% relativer Feuchte eine Masse-Schicht von 80-100 $g/m^2$ übrig blieb.

Diese Schicht wurde sodann auf ein Textil-Gewebe mit einem Gewicht von 120 $g/m^2$ überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 6

15 Gewichtsteile Gelatine (enthaltend 10 Gew.-% Wasser), 8 Gewichtsteile Polyethylenglycol 600 , 5 Gewichtsteile $CaCl_2$ (enthaltend 2 Gew.-% Wasser) wurden in 71,5 Gewichtsteile Wasser gegeben und sodann unter Rühren auf 60 ° C gebracht bis eine homogene Lösung entstanden war. In diese Lösung wurden 0,5 Gewichtsteile 3-Aminopropyltrisethoxysilan hineingerührt. Diese Lösung wurde auf eine ebene polierte Teflonplatte gegossen, so daß nach 48 Stunden bei 22° C und 40% relativer Feuchte eine Masse-Schicht von 80-100 g/m² übrig blieb.

Diese Schicht wurde sodann auf ein Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 7

15 Gewichtsteile Gelatine (enthaltend 10 Gew.-% Wasser), 10 Gewichtsteile Polyethylenglycol 1020, 6 Gewichtsteile $CaCl_2$ (enthaltend 2 Gew.-% Wasser) wurden in 68,5 Gewichtsteile Wasser gegeben und sodann unter Rühren auf 60° C gebracht bis eine homogene Lösung entstanden war. In diese Lösung wurden 0,5 Gewichtsteile 3-Aminopropyltrisethoxysilan hineingerührt. Diese Lösung wurde auf eine ebene polierte Teflonplatte gegossen, so daß nach 48 Stunden bei 22° C und 40% relativer Feuchte eine Masse-Schicht von 100-120 g/m² übrig blieb.

Diese Schicht wurde sodann auf ein Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 8

15 Gewichtsteile Gelatine (enthaltend 10 Gew.-% Wasser), 10 Gewichtsteile Glycerin (enthaltend 15 Gew.-% Wasser), 10 Gewichtsteile $ZnCl_2$ (enthaltend 2 Gew.-% Wasser) wurden in 64,8 Gewichtsteile Wasser gegeben und sodann unter Rühren auf 55° C gebracht bis eine homogene Lösung entstanden war. In diese Lösung wurden 0,2 Gewichtsteile 3-Aminopropyltrisethoxysilan hineingerührt. Diese Lösung wurde auf eine ebene polierte Teflonplatte gegcssen, so daß nach 48 Stunden bei 22° C und 40% relativer Feuchte eine Masse-Schicht von 100 - 120 g/m² übrig blieb.

Diese Schicht wurde sodann auf ein Textil-Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 9

15 Gewichtsteile Gelatine (enthaltend 10 Gew.-% Wasser), 10 Gewichtsteile Glyzerin (enthaltend 15 Gew.-% Wasser), 6 Gewichtsteile $ZnCl_2$ (enthaltend 2 Gew.-% Wasser) wurden in 68,8 Gewichtsteile Wasser gegeben und sodann unter Rühren auf 55° C gebracht bis eine homogene Lösung entstanden war. In diese Lösung wurden 0,2 Gewichtsteile 3-Aminopropyl-trisethoxysilan hineingerührt. Diese Lösung wurde auf eine ebene polierte Teflonplatte gegossen, so daß nach 48 Stunden bei 22° C und 40% relativer Feuchte eine Masse-Schicht von 100-120 g/m² übrig blieb.

Diese Schicht wurde sodann auf ein Textil-Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 10

15 Gewichtsteile Gelatine (enthaltend 10 Gew.-% Wasser), 10 Gewichtsteile Propylenglycol-1,2 , 5 Gewichtsteile $ZnCl_2$ (enthaltend 2 Gew.-% Wasser) wurden in 69,7 Gewichtsteile Wasser gegeben und sodann unter Rühren auf 60° C gebracht bis eine homogene Lösung entstanden war. In diese Lösung wurden 0,3 Gewichtsteile 3-Aminopropyl-trismethoxysilan gerührt. Diese Lösung wurde auf eine ebene polierte Teflonplatte gegossen, so daß nach 48 Stunden bei 22° C und 40% relativer Feuchte eine Masse-Schicht von 100-120 g/m² übrig blieb.

Diese Schicht wurde sodann auf ein Textil-Gewebe mit einem Gewicht von 100 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 11

15 Gewichtsteile Gelatine (enthaltend 10 Gew.-% Wasser), 10 Gewichtsteile Glyzerin (enthaltend 15 Gew.-% Wasser), 5 Gewichtsteile $CaCl_2$ (enthaltend 2 Gew.-% Wasser) wurden in 69,8 Gewichtsteile Wasser gegeben und sodann unter Rühren auf 60° C gebracht bis eine homogene Lösung entstanden war. In diese Lösung wurden 0,2 Gewichtsteile Methyltrimethoxysilan hineingerührt. Diese Lösung wurde auf eine ebene polierte Teflonplatte gegossen, so daß nach 48 Stunden bei 22° C und 40% relativer Feuchte eine Masse-Schicht von 100-120 g/m² übrig blieb.

Diese Schicht wurde sodann auf ein Textil-Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

Beispiel 12

20 Gewichtsteile Gelatine (enthaltend 10 Gew.-% Wasser), 6 Gewichtsteile Ethylendiglycol 400 , 6 Gewichtsteile $ZnCl_2$ (enthaltend 2 Gew.-% Wasser) wurden in 67,8 Gewichtsteile Wasser gegeben und sodann unter Rühren auf 55° C gebracht bis eine homogene Lösung entstanden war. In diese Lösung wurden 0,2 Gewichtsteile Methyltrimethoxysilan hineingerührt. Diese Lösung wurde auf eine ebene polierte Teflonplatte gegossen, so daß nach 48 Stunden bei 22° C und 40% relativer Feuchte eine Masse-Schicht von 80-100 g/m² übrig blieb.

Diese Schicht wurde sodann auf ein Textil-Gewebe mit einem Gewicht von 120 g/m² überkaschiert und die Selbstklebeschicht mit einer geriffelten PVC-Abdeckfolie geschützt. Dieses abgedeckte Pflaster wurde in Streifen von 3cm Länge und 0,8 cm Breite geschnitten und diese Streifen nach Abziehen der Abdeckfolie auf verschiedene Hautpartien geklebt: Handrücken, Finger, Rücken, Oberarm-Innenseite und Bauch und 24 Stunden dort belassen und sodann abgezogen. Alle Pflaster ließen sich ohne Rückstände wieder abziehen, die entsprechenden Hautpartien zeigten keine klebende Rötung oder andere Beanspruchungen.

**Patentansprüche**

1. Druckempfindliche Selbstklebemasse, enthaltend vernetzte Gelatine, einen organischen Weichmacher und $CaCl_2$ und/oder $ZnCl_2$.

2. Selbstklebemasse nach Anspruch 1, dadurch gekennzeichnet, daß die Gelatine mit einem Vernetzer, gewählt aus der Gruppe der
   Alkoxysilylverbindungen, bevorzugt aus der Gruppe der Alkoxysilylverbindungen der folgenden allgemeinen Formel:
   $HRN- (CH_2-CH_2-NR')_n-(CH_2)_m-Si(OR'')_3$
   mit R, R' gewählt aus der Gruppe H, Methyl oder Ethyl,
   und R'' gewählt aus der Gruppe Methyl, Ethyl
   wobei n Werte von 0 - 5 und
   m Werte von 1 - 4 annehmen kann,
   vernetzt wird.

3. Selbstklebemasse nach Anspruch 1 oder 2, dadurch gekennzeichnet, daß der Vernetzer gewählt wird aus der Gruppe

3-Aminopropyltriethoxysilan,

N-Aminoethylaminopropyltrimethoxysilan, "Triaminosilan" ($H_2N-CH_2-CH_2-NH-CH_2-CH_2-NH-CH_2-CH_2-CH_2-Si(OCH_3)_3$),

N-Methyl-3-aminopropyltrimethoxysilan,

3-Ureidopropyltriethoxysilan,

3-(4,5-Dihydroimidazol-1-yl)-propyltriethoxysilan,

3-Methacryloxypropyltriethoxysilan,

3-Glycidyloxypropyltrimethoxysilan, Vinyltriethoxysilan,

Vinyltrimethoxysilan, Tetramethoxysilan oder Tetraethoxysilan.

4. Selbstklebemasse nach einem der Ansprüche 1 - 3, dadurch gekennzeichnet, daß der Vernetzer aus der Gruppe der mehrfunktionellen Isocyanate gewählt wird, und daß der Vernetzer gegebenenfalls zusammen mit einem Emulgator zugegeben wird.

5. Selbstklebemasse nach einem der Ansprüche 1 - 4, dadurch gekennzeichnet, daß der Weichmacher gewählt wird aus der Gruppe
Ethylenglycol, flüssige Polyethylenglycole verschiedener Molmasse, Propylenglycol-1,2, flüssige Polypropylenglycole-1,2 verschiedener Molmasse, Propylenglycol-1,3, flüssige Polypropylenglycole-1,3 verschiedener Molmasse, Zitronensäuretriethyl- oder trimethylester, Milchsäuremethyl- oder -ethylester, Glycolsäuremethyl- oder -ethylester, 2-Ethyl-2(hydroxymethyl)-1,3-propandiol oder der verzweigten Polyole.

6. Selbstklebemasse nach einem der Ansprüche 1 - 5, enthaltend

$$25 - 50 \quad \text{Gew.-\% Gelatine}$$

$$15 - 45 \quad \text{Gew.-\% Weichmacher}$$

$$10 - 40 \quad \text{Gew.-\% CaCl}_2 \text{ und/oder ZnCl}_2$$

$$0,1 - 5,0 \quad \text{Gew.-\% Vernetzer,}$$

bezogen auf das Trockengewicht der Gesamtzusammensetzung.

7. Selbstklebemasse nach einem der Ansprüche 1 - 6, enthaltend

$$25 - 50 \quad \text{Gew.-\% Gelatine}$$

$$15 - 45 \quad \text{Gew.-\% Weichmacher}$$

$$10 - 40 \quad \text{Gew.-\% CaCl}_2 \text{ und/oder ZnCl}_2$$

$$0,1 - 5,0 \quad \text{Gew.-\% Vernetzer}$$

$$0,001 - 10,0 \quad \text{Gew.-\% Emulgator,}$$

bezogen auf das Trockengewicht der Gesamtzusammensetzung.

8. Verfahren zur Herstellung einer Selbstklebemasse nach einem der Ansprüche 1 - 7, dadurch gekennzeichnet, daß zunächst die Gelatine unter Rühren bei 45 - 95 °C in Wasser gelöst wird, sodann die anderen Bestandteile in beliebiger Reihenfolge und der Vernetzer zuletzt dazugegeben werden.

9. Selbstklebende Artikel in Bahn-, Folien- Blatt- oder Bandform, dadurch erhältlich, daß eine Selbstklebemasse nach einem der Ansprüche 1 - 7 bei 45 - 95 °C auf einen flächigen Träger, gewählt aus der Gruppe der Gewebe (wie Baumwolle, Polyestergewebe, Polyamidgewebe, Zellwollgewebe), Vliese (wie Polyethylenvlies), Papier oder Folien (wie Polyesterfolie, PVC-Folie oder sonstige Hart- oder Weichfolien) aufgetragen wird.

10. Verwendung eines selbstklebenden Artikels nach Anspruch 9 als medizinisches Pflaster, als Etikett oder als Klebeband.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | PATENT ABSTRACTS OF JAPAN, Band 12, Nr. 297 (C-519)[3144], 12. August 1988, Seite 112; & JP-A-63 66 120 (NIPPON OIL & FATS) 24-03-1988 * Zusammenfassung * — — — | 1,5 | C 09 J 189/00 A 61 L 15/32 |
| Y | IDEM — — — | 1,9,10 | |
| Y | US-A-3 249 109  (H. MAETH et al.) * Spalte 1, Zeilen 25-46; Spalte 4, Anspruch 1 * — — — | 1,9,10 | |
| A | GB-A-1 127 625  (JOHNSON & JOHNSON) * Seite 1, Zeilen 69-76 * — — — | 1,2,3 | |
| A | PATENT ABSTRACTS OF JAPAN, Band 11, Nr. 232 (C-437)[2679], 29. Juli 1987, Seite 128; & JP-A-62 45 678 (TAKIRON) 27-02-1987 * Zusammenfassung * — — — | 1 | |
| A | PATENT ABSTRACTS OF JAPAN, Band 12, Nr. 438 (C-544)[3285], 17. November 1988, Seite 55; & JP-A-63 162 610 (NITTA ZERACHIN) 06-07-1988 * Zusammenfassung * — — — — — | 1 | RECHERCHIERTE SACHGEBIETE (Int. Cl.5) C 09 J C 08 K A 61 L C 09 H |

Der vorliegende Recherchenbericht wurde für alle Patentansprüche erstellt

| Recherchenort | Abschlußdatum der Recherche | Prüfer |
|---|---|---|
| Den Haag | 22 April 91 | MAZET J.-F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument